# EUROPÄISCHE PATENTSCHRIFT

(11) **EP 3 360 020 B1**
(45) Veröffentlichungstag und Bekanntmachung des Hinweises auf die Patenterteilung: **27.01.2021**
(21) Anmeldenummer: 16775255.9
(22) Anmeldetag: 04.10.2016
(51) Int. Cl.: G05B 19/423

(54) **ROBOTERARM**
ROBOT ARM
BRAS DE ROBOT

(30) Priorität: 08.10.2015 DE 102015117213
(43) Veröffentlichungstag der Anmeldung: 15.08.2018
(73) Patentinhaber: Kastanienbaum GmbH, 80538 München (DE)
(72) Erfinder: HADDADIN, Sami, 30173 Hannover (DE)
(74) Vertreter: Rösler Rasch van der Heide & Partner
(86) Internationale Anmeldenummer: PCT/EP2016/073621
(87) Internationale Veröffentlichungsnummer: WO 2017/060212

(56) Entgegenhaltungen:
- EP-A2- 0 108 348
- DE-A1-102014 204 452
- JP-U- S58 160 666
- US-A1- 2009 259 412

## Beschreibung

Die Erfindung betrifft einen Roboterarm mit einer Anzahl N von Armgliedern Aₙ, die über eine Anzahl N aktorisch antreibbare Gelenkverbindungen GVₙ mit einem Roboterkörper verbindbar sind. Die Erfindung betrifft darüber hinaus einen Roboter mit einem ebensolchen Roboterarm.

Bei einer Zusammenarbeit von Mensch und Roboter ist insbesondere eine sogenannte Lernprogrammierung ("Teach-in" Verfahren) des Roboters bekannt. Hierbei ergreift der Mensch einen Roboterarm des Roboters, insbesondere am Endeffektor, und führt eine zu lernende Bewegung des Roboterarms aus. Dabei kompensiert der Roboter vorteilhaft die Trägheit und das Eigengewicht des Roboterarms. Die ausgeführte Bewegung des Roboterarms wird gespeichert und kann nachfolgend automatisch vom Roboter durchgeführt werden. Dies ermöglicht eine einfache und schnelle Programmierung des Roboters. Natürlich ist eine Nachbearbeitung bzw. Optimierung der ausgeführten und gespeicherten Bewegung des Roboterarms möglich.

Bei diesem "Teach-in" Verfahren muss der Roboterarm aus verschiedenen Positionen gegriffen und geführt werden. Weiterhin sind Bedienelemente und gegebenenfalls Anzeigeelemente am Roboter bzw. am Roboterarm erforderlich, um die Lernprogrammierung zu steuern bzw. Statusanzeigen oder sonstige Ausgaben zu ermöglichen.

Im Stand der Technik sind Roboter mit Roboterarmen bekannt, die zur Lernprogrammierung Bedienelemente am Roboterarm aufweisen. Derartige Roboter bzw. Roboterarme gehen beispielsweise aus folgenden Dokumenten hervor: EP 0 108 348 A2, JP S58 160666U, US 2009/259412 A1 und DE10 2014 204452 A1.

Die Aufgabe der Erfindung ist es, einen Roboterarm anzugeben, der eine verbesserte Lernprogrammierung eines Roboters mit einem Roboterarm ermöglicht.

Die Erfindung ergibt sich aus den Merkmalen der unabhängigen Ansprüche. Vorteilhafte Weiterbildungen und Ausgestaltungen sind Gegenstand der abhängigen Ansprüche. Weitere Merkmale, Anwendungsmöglichkeiten und Vorteile der Erfindung ergeben sich aus der nachfolgenden Beschreibung, sowie der Erläuterung von Ausführungsbeispielen der Erfindung, die in den Figuren dargestellt sind.

Ein erster Aspekt der Erfindung betrifft einen Roboterarm, der eine Anzahl N von Armgliedern Aₙ aufweist, die über eine Anzahl N aktorisch antreibbare Gelenkverbindungen GVₙ mit einem Roboterkörper verbindbar sind, mit n= 1, 2, ..., N.

Der vorgeschlagene Roboterarm zeichnet sich erfindungsgemäß dadurch aus, dass das distale Armglied A_{N} des Roboterarms an seinem proximalen Ende über die Gelenkverbindung GV_{N} mit dem Armglied A_{N-1} verbunden ist, das proximale Armglied A₁ des Roboterarms an seinem proximalen Ende über die Gelenkverbindung GV₁ mit dem Roboterkörper verbindbar ist, die Gelenkverbindung GV_{N} eine Drehung des Armglieds A_{N} um eine Drehachse D_{N} ermöglicht, das Armglied A_{N} des Roboterarms sich entlang einer Achse L1 erstreckt und die Achse L1 mit der Drehachse D_{N} einen Winkel von 50 bis 130° einschließt, das distale Ende des Armglieds A_{N} über eine aktorisch antreibbare Gelenkverbindung GV_{N+1} mit einem Effektor E verbindbar ist, wobei die Gelenkverbindung GV_{N+1} eine Drehung des Effektors E um eine Drehachse D_{N+1} ermöglicht. Weiterhin erfindungsgemäß schließen die Drehachsen D_{N} und D_{N+1} einen Winkel W1 im Bereich von 50 bis 130° ein, und die Drehachse D_{N+1} und die Achse L1 einen Winkel W2 im Bereich von 50 bis 130° ein, wobei an dem Effektor E oder an einem zwischen der Gelenkverbindung GV_{N+1} und dem Effektor E angeordneten Zwischenstück ZW ein sich senkrecht zur Drehachse D_{N+1} erstreckender, mit einer Hand umgreifbarer und damit (mit dem Effektor E oder der Gelenksverbindung GV_{N+1} oder dem Armglied A_{N}) starr verbundener Fortsatz F2 ausgebildet ist. Weiterhin erfindungsgemäß zeichnet sich der Roboterarm dadurch aus, dass das Armglied A_{N} einen damit (dem Armglied A_{N}) starr verbundenen griffartigen Fortsatz F1 aufweist, der sich konzentrisch zur Drehachse D_{N+1} erstreckt, ein freies Ende FE des Fortsatzes gegenüber dem Rest des Fortsatzes F1 um die Drehachse D_{N+1} drehbar ist oder um die Drehachse D_{N+1} rotierbar anordenbar ist, und das freie Ende FE Eingabeelemente EE zur manuellen Eingabe von Daten aufweist.

Der vorgeschlagene Roboterarm ermöglicht insbesondere eine feinfühlige, sensible und genauere Führung und Bewegung des Roboterarms durch einen Bediener, und somit eine genauere Lernprogrammierung eines Roboters mit einem ebensolchen Roboterarm. Insbesondere können Kräfte und Momente über den Fortsatz F2 feinfühliger und genauer bei der Lernprogrammierung vorgegeben werden.

Die vorgeschlagene Anordnung und Ausführung der Eingabeelemente EE am freien Ende FE des Fortsatzes ermöglicht insbesondere eine gute ergonomische Bedienung, insbesondere bei einer Lernprogrammierung, eines Roboters mit einem solchen Roboterarm. Die Eingabe von Informationen über die Eingabeelemente EE erfolgt vorteilhaft manuell, d.h. mittels Berührung bzw. manueller Bedienung der Eingabeelemente EE.

Eine vorteilhafte Weiterbildung zeichnet sich dadurch aus, dass an dem Zwischenstück ZW ein oder mehrere Eingabeelemente EE_{zw} in der Reichweite eines Daumens der den Fortsatz F2 greifenden Hand angeordnet sind. Die Eingabeelemente EE_{zw} sind vorzugsweise derart angeordnet, dass eine bequeme und ergonomische Bedienung der Eingabeelemente EE_{zw} möglich ist.

Vorteilhaft sind die Eingabeelemente EE am freien Ende FE zumindest teilweise konzentrisch um die Drehachse D_{N+1} angeordnet. Das heißt, dass zwei, drei, ... oder alle der Eingabeelemente EE konzentrisch um die Drehachse D_{N+1}, vorteilhaft mit einem identischen Radius zur Drehachse D_{N+}1, angeordnet sind. Wird der Fortsatz F1 mit einer Hand umgriffen und werden die Eingabeelemente EE mit dem Daumen der Hand bedient, so sind die Eingabeelemente EE für den Daumen somit auch bei unterschiedlichen Ausrichtungen der Hand gleichbleibend gut erreich- bzw. bedienbar.

Vorteilhaft ist ein Eingabeelement EE₀ der Eingabeelemente EE axial zur Drehachse D_{N+1} angeordnet. Dieses zentrale Eingabeelement EE₀ dient vorteilhaft zur Eingabe oder Steuerung aktuell primärer Parameter, beispielsweise zur Eingabe eines Startzeitpunktes und eines Endzeitpunktes einer Lernprogrammierung.

Vorteilhaft können die über die Eingabeelemente EE bzw. EE_{zw} jeweils eingegebenen oder gesteuerten Parameter automatisch oder manuell abhängig von der jeweiligen Aufgabenstellung variabel zugeordnet bzw. ausgewählt werden.

Vorteilhaft ist der Fortsatz F1 und/oder der Fortsatz F2 derart ausgebildet, dass er von einer Hand eines Bedieners umgriffen werden kann, wobei ein Daumen der Hand auf dem freien Ende FE zur Bedienung der Eingabeelemente EE bzw. auf den Eingabeelementen EE_{zw} zu liegen kommt. Hierzu weist der Fortsatz F1 bzw. der Fortsatz F2 vorteilhaft eine Zylinderform oder eine ergonomische Griffform auf.

Vorteilhaft ist das freie Ende FE des Fortsatzes F1 zur Bedienung der Eingabeelemente EE mit dem Daumen der Hand ergonomisch ausgebildet. Hierzu sind in einer

Ausführungsform die Eingabeelemente EE auf einer Fläche angeordnet, die gegenüber einer Fläche senkrecht zur Drehachse D_{N+1} in einem Winkel von 5 bis 60° geneigt ist. Durch diese Neigung ist eine ergonomische Bedienung der Eingabeelemente EE möglich, wobei das distale Daumengelenk der bedienenden Hand insbesondere nicht beispielsweise um 90° abgewinkelt werden muss.

Vorteilhaft weisen die Eingabeelemente EE und/oder die Eingabeelemente EE_{zw} Leuchtelemente mit einer oder mehreren ansteuerbaren Lichtquellen auf. Die Lichtquelle/n sind vorteilhaft Leuchtdioden LEDs.

Die Eingabeelemente EE bzw. EEzw sind vorteilhaft als Taster und/oder Wipptaster und/oder Druckknöpfe und/oder Schalter und/oder Drehknöpfe, und/oder Schieberegler ausgeführt. In einer weiteren vorteilhaften Ausführungsform sind die Eingabeelemente EE als Eingabefelder eines Touchpads (berührempfindliche Fläche) oder eines Touchscreens (berührempfindliches Display) ausgeführt.

In einer Weiterbildung des vorgeschlagenen Roboterarms ist das freie Ende FE des Fortsatzes F1 mit dem Rest des Fortsatzes F1 in verschiedenen Ausrichtungen um die Drehachse D_{N+1} lösbar fest verbindbar ist (bspw. mittel Rast- oder Klippverbindungen). Das heißt, dass das freie Ende FE vom mit dem Roboterarm starr verbundenen Rest des Fortsatzes F1 lösbar ist, und in einer anderen, gegenüber der Drehachse D_{N+1} rotierten Lage wieder mit dem Rest des Fortsatzes F1 verwendbar ist. So können die Eingabeelemente EE in ihrer Ausrichtung relativ zu der den Fortsatz F1 ergreifenden Hand jeweils optimal ausgerichtet werden. Allerdings erfordert diese Ausrichtung jeweils ein Lösen des freien Endes FE und ein erneutes Verbinden des freien Endes FE mit dem Rest des Fortsatzes F1 in einer neuen Winkelorientierung.

In einer besonders vorteilhaften Ausführungsform des Roboterarms ist das freie Ende FE des Fortsatzes F1 um die Drehachse D_{N+1} rotierbar fest mit dem Rest des Fortsatzes verbunden. In dieser Ausführungsform kann das freie Ende FE vorteilhaft frei (d.h. ohne Anschlag) um die Drehachse D_{N+1} rotiert werden. Das Lösen und erneute Verbinden des freien Endes FE, wie in der vorangehenden Weiterbildung ausgeführt, entfällt in dieser Ausführungsform.

Eine Weiterbildung zeichnet sich dadurch aus, dass zwischen dem freien Ende FE und dem Rest des Fortsatzes F1 eine mechanische und/oder elektrische und/oder eine magnetische Rastervorrichtung vorhanden ist, die ein Rotieren des freien Endes FE mit einer vorgegebenen Rasterung um die Drehachse D_{N+1} erlaubt. Dies verhindert insbesondere ein ungewolltes Verdrehen des freien Endes FE gegenüber dem Rest des Fortsatzes F1. Um von einer Rasterstellung in eine benachbarte Rasterstellung zu gelangen, ist es erforderlich, eine vorgegebene Kraft bzw. ein vorgegebenes Drehmoment auf das freie Ende FE aufzubringen. Vorteilhaft wird diese Kraft bzw. dieses Drehmoment derart gewählt, dass eine ergonomische Bedienung der Eingabeelemente bei unterschiedlichen Angriffsrichtungen der Hand am Fortsatz F1 möglich ist. Vorteilhaft lässt sich diese Kraft bzw. dieses Drehmoment variabel je nach Bedarf vorgeben.

Eine Weiterbildung zeichnet sich dadurch aus, dass an dem Armglied A_{N} eine Ausgabeeinheit zur Ausgabe von graphischen und/oder alphanumerischen Informationen angeordnet ist. Dies ermöglicht die Anzeige bzw. Ausgabe von relevanten Informationen beispielsweise während der Ausführung einer Lernprogrammierung.

Eine Weiterbildung zeichnet sich dadurch aus, dass der Roboterarm bzw. insbesondere das Armglied A_{N} eine Schnittstelle aufweist, über die als Ausgabeeinheit ein Smartphone anschließbar ist. Diese Schnittstelle kann eine mechanische/elektrische Schnittstelle oder eine Funkschnittstelle (beispielsweise Bluetooth, WLAN etc.).

Der Fortsatz F1 und der Fortsatz F2 werden vorteilhaft jeweils von einer Hand eine Bedienperson gegriffen bzw. geführt.

In einer besonders bevorzugten Ausführungsform werden für die eingangs genannten Winkel W1 = 90° und/oder W2 = 90° gewählt.

Ein weiterer Aspekt der Erfindung betrifft einen Roboter mit einem Roboterarm wie vorstehend beschrieben.

Vorteilhaft umfasst der Roboter Sensoren zur Erfassung eines mechanischen Zustandes Z(t) des Roboterarms, eine Einheit zur Erfassung von Eingaben EG(t) über die Eingabeelemente EE und/oder EEzw, eine Einheit zur Auswertung der Zustände Z(t) und der Eingaben EG(t) zur Ermittlung von Auswertergebnissen AW(t), und eine Speichereinheit zur Speicherung der Auswerteergebnisse AW(t).

Vorteilhaft sind die Auswertergebnisse AW(t) Steueranweisungen zur Ansteuerung des Roboterarms. Der vorgeschlagene Roboter ermöglicht somit die Erfassung von Zuständen Z(t) des Roboterarms, die insbesondere durch die mechanische Führung eines Bedieners erzeugt werden, die Erfassung von Eingaben des Bedieners über die Eingabeelemente EE und/oder EE_{zw}, die Auswertung der erfassten Zustände Z(t) und Eingaben und deren Umsetzung in Auswerteergebnisse AW(t), die insbesondere Steueranweisungen darstellen, um die eingegebenen Bewegungen des Roboterarms auszuführen.

Unter dem mechanischen Zustand Z(t) wird vorliegend vorteilhaft die Positionierung des Roboterarms, zeitliche Ableitung/en der Positionierung des Roboterarms und/oder auftretende und einwirkende Kräfte und Momente auf den Roboterarm verstanden. Natürlich kann der Zustand Z(t) weitere zeitabhängige Zustandsparameter je nach Aufgabenstellung und Anforderung umfassen. Die Einheit zur Erfassung der Eingaben EG(t) umfasst vorteilhaft die Eingabeelemente EE und/oder EE_{zw} sowie eine mit den Eingabeelementen verbundene Prozessoreinheit PE1. Die Einheit zur Auswertung der Zustände Z(t) ist vorteilhaft ein Computer bzw. eine Prozessoreinheit PE2, durch den/die ein entsprechendes Auswerteprogramm ausgeführt wird. Vorteilhaft sind die Prozessoreinheiten PE1 und PE2 identisch.

Weitere Vorteile, Merkmale und Einzelheiten ergeben sich aus der nachfolgenden Beschreibung, in der - gegebenenfalls unter Bezug auf die Zeichnung - zumindest ein Ausführungsbeispiel im Einzelnen beschrieben ist. Es zeigt:

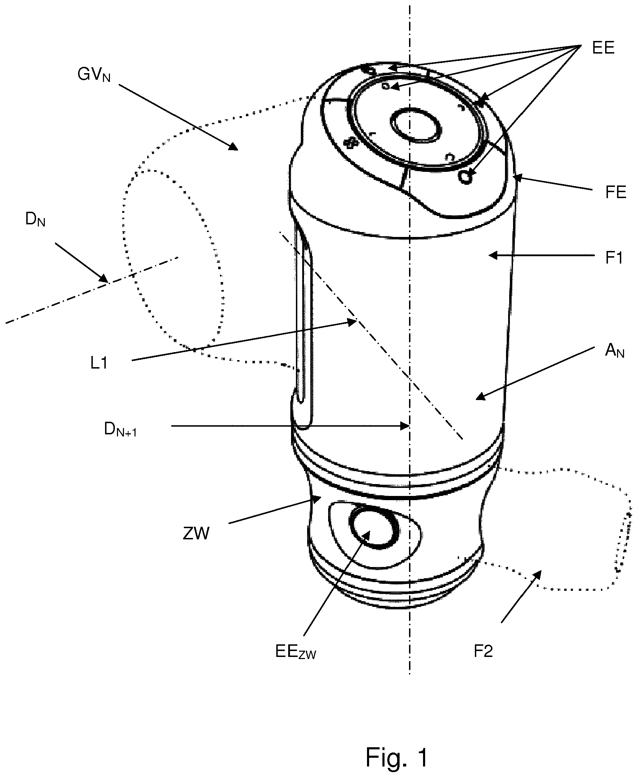

- Fig. 1: eine schematisierte Teildarstellung eines Ausführungsbeispiels des vorgeschlagenen Roboterarms.

**Fig. 1** zeigt eine schematisierte Darstellung eines Ausführungsbeispiels des vorgeschlagenen Roboterarms. Der in Teilen dargestellte Roboterarm weist eine Anzahl N von Armgliedern Aₙ auf, die über eine Anzahl N aktorisch antreibbare Gelenkverbindungen GVₙ mit einem Roboterkörper verbindbar sind, mit n= 1, 2, ..., N. In Fig. 1 ist lediglich das Armglied A_{N-1} und A_{N} dargestellt. Der in Teilen dargestellte Roboterarm zeichnet sich dadurch aus, dass das distale Armglied A_{N} des Roboterarms an seinem proximalen Ende über die Gelenkverbindung GV_{N} mit dem Armglied A_{N-1} verbunden ist, das proximale Armglied A₁ an seinem proximalen Ende über die Gelenkverbindung GV₁ mit dem Roboterkörper (nicht dargestellt) verbindbar ist, die Gelenkverbindung GV_{N} eine Drehung des Armglieds A_{N} um eine Drehachse D_{N} ermöglicht, das Armglied A_{N} des Roboterarms sich entlang einer Achse L1 erstreckt und die Achse L1 mit der Drehachse D_{N} einen Winkel von 90° einschließt, das distale Ende des Armglieds A_{N} über eine aktorisch antreibbare Gelenkverbindung GV_{N+1} mit einem Effektor E (nicht dargestellt) verbindbar ist, wobei die Gelenkverbindung GV_{N+1} eine Drehung des Effektors E um eine Drehachse D_{N+1} ermöglicht, wobei die Drehachsen D_{N} und D_{N+1} einen Winkel W1 im Bereich von 90° einschließen, und wobei die Drehachse D_{N+1} und die Achse L1 einen Winkel W2 von 90° einschließen.

Der in Teilen dargestellte Roboterarm zeichnet sich weiterhin dadurch aus, dass an einem zwischen der Gelenkverbindung GV_{N+1} und dem Effektor E (nicht dargestellt) angeordneten Zwischenstück ZW ein sich senkrecht zur Drehachse D_{N+1} d.h. radial erstreckender, mit einer Hand umgreifbarer und damit starr verbundener Fortsatz F2 ausgebildet ist. Weiterhin weist das Armglied A_{N} einen mit dem Armglied A_{N} starr verbundenen griffartigen Fortsatz F1 auf, der sich konzentrisch zur Drehachse D_{N+1} erstreckt, wobei ein freies Ende FE des Fortsatzes F1 gegenüber dem Rest des Fortsatzes F1 um die Drehachse D_{N+1} frei rotierbar ist, und das freie Ende FE Eingabeelemente EE zur manuellen Eingabe von Daten aufweist.

Auf der Oberseite des freien drehbaren Endes FE sind vier axial angeordnete Eingabeelemente EE, ein zentral axial angeordnetes Eingabeelement EE, sowie auf abgeschreckten Flächen insgesamt vier weitere Eingabeelemente EE angeordnet. Die elektrische Übertragung von Signalen der Eingabeelemente EE zwischen dem lösbaren Ende FE und dem Rest des Fortsatzes F1 erfolgt vorteilhaft drahtgebunden, oder mittels einer Schleifringübertragung, oder mittels einer Funkübertragung.

Vorteilhaft umfassen die Eingabeelemente EE zumindest teilweise Leuchtelemente mittels denen beispielsweise ein aktueller Eingabestatus ausgegeben werden kann und/oder die zur Kenntlichmachung aktuell aktiver Eingabeelemente EE dienen, und/oder die einfach zur Beleuchtung der Eingabeelemente EE dienen. Weiterhin vorteilhaft weist der Roboterarm eine akustische und/oder optische Ausgabeeinheit auf, über die ein in Zusammenhang mit einer Bedienung eines Eingabeelements EE erzeugter Status akustisch und oder optisch ausgebbar ist. So kann über die Ausgabeeinheit ein Roboterstatus oder Fehlermeldungen ausgegeben werden.

Durch die flexible Anordnung des lösbaren freien Endes FE ist eine für den Bedienter passende Ausrichtung möglich, um Eingaben aus verschiedenen Blickwinkeln und Greifrichtungen zu ermöglichen.

Der Fortsatz F2 ermöglicht insbesondere eine sensible und genaue Positionierung und Vorgabe von Kräften oder Momenten während des sogenannten "Teach-in" Vorgangs.

Vorteilhaft erfolgt der "Teach-in" Vorgang indem eine Hand eines Bedieners den Fortsatz F1 und die andere Hand den Fortsatz F2 greift, und erforderliche Eingaben in die Eingabeelemente EE bzw. EEzw mittels der jeweiligen Daumen erfolgen.

Obwohl die Erfindung im Detail durch bevorzugte Ausführungsbeispiele näher illustriert und erläutert wurde, so ist die Erfindung nicht durch die offenbarten Beispiele eingeschränkt und andere Variationen können vom Fachmann hieraus abgeleitet werden, ohne den Schutzumfang der Erfindung zu verlassen. Es ist daher klar, dass eine Vielzahl von Variationsmöglichkeiten existiert. Es ist ebenfalls klar, dass beispielhaft genannte Ausführungsformen wirklich nur Beispiele darstellen, die nicht in irgendeiner Weise als Begrenzung etwa des Schutzbereichs, der Anwendungsmöglichkeiten oder der Konfiguration der Erfindung aufzufassen sind. Vielmehr versetzen die vorhergehende Beschreibung und die Figurenbeschreibung den Fachmann in die Lage, die beispielhaften Ausführungsformen konkret umzusetzen, wobei der Fachmann in Kenntnis des offenbarten Erfindungsgedankens vielfältige Änderungen beispielsweise hinsichtlich der Funktion oder der Anordnung einzelner, in einer beispielhaften Ausführungsform genannter Elemente vornehmen kann, ohne den Schutzbereich zu verlassen, der durch die Ansprüche und deren rechtliche Entsprechungen, wie etwa weitergehenden Erläuterung in der Beschreibung, definiert wird.

## Patentansprüche

1. Roboterarm mit einer Anzahl (N) von Armgliedern (Aₙ) die über die gleiche Anzahl (N) von aktorisch antreibbaren Gelenkverbindungen (GVₙ) mit einem Roboterkörper verbindbar sind, mit n= 1, 2, ..., N; wobei
- das distale Armglied (A_{N}) des Roboterarms an seinem proximalen Ende über die distale Gelenkverbindung (GV_{N}) mit dem vorigen Armglied (A_{N-1}) verbunden ist,
- das proximale Armglied (A₁) an seinem proximalen Ende über die proximale Gelenkverbindung (GV₁) mit dem Roboterkörper verbindbar ist,
- die distale Gelenkverbindung (GV_{N}) eine Drehung des distalen Armglieds (A_{N}) um eine Drehachse (D_{N}) ermöglicht,
- das distale Armglied (A_{N}) des Roboterarms sich entlang einer Achse (L1) erstreckt, welche Achse (L1) mit der Drehachse (D_{N}) des distalen Armglieds (A_{N}) einen Winkel von 50 bis 130° einschließt,
- das distale Ende des distalen Armglieds (A_{N}) über eine zusätzliche aktorisch antreibbare Gelenkverbindung (GV_{N+1}) mit einem Effektor (E) verbindbar ist, wobei die zusätzliche Gelenkverbindung (GV_{N+1}) eine Drehung des Effektors (E) um eine Drehachse (D_{N+1}) ermöglicht, wobei
∘ die Drehachse (D_{N}) des distalen Armglieds (A_{N}) und die Drehachse (D_{N+1}) des Endeffektors (E) einen Winkel (W1) im Bereich von 50 bis 130° einschließen,
∘ die Drehachse (D_{N+1}) des Endeffektors (E) und die Achse (L1) des distalen Armglieds (A_{N}) einen Winkel (W2) im Bereich von 50 bis 130° einschließen,
∘ an dem Effektor (E) oder an einem zwischen der Gelenkverbindung (GV_{N+1}) und dem Effektor (E) angeordneten Zwischenstück (ZW) oder an dem distalen Armglied (A_{N}) ein sich senkrecht zur Drehachse (D_{N+1}) des Endeffektors (E) erstreckender, mit einer Hand umgreifbarer und damit starr verbundener Fortsatz (F2) ausgebildet ist, und
∘ das distale Armglied (A_{N}) einen damit starr verbundenen griffartigen zweiten Fortsatz (F1) aufweist, der sich konzentrisch zur Drehachse (D_{N+1}) des Endeffektors (E) erstreckt, wobei ein freies Ende (FE) des zweiten Fortsatzes (F1) gegenüber dem Rest des zweiten Fortsatzes (F1) um die Drehachse (D_{N+1}) des Endeffektors (E) drehbar ist oder um die Drehachse (D_{N+1}) des Endeffektors rotierbar anordenbar ist, und wobei das freie Ende (FE) des zweiten Fortsatzes (F1) Eingabeelemente (EE) zur manuellen Eingabe von Daten aufweist.

2. Roboterarm nach Anspruch 1,
bei dem an dem Zwischenstück (ZW) ein oder mehrere Eingabeelemente (EE_{ZW}) in der Reichweite eines Daumens der Hand angeordnet sind.

3. Roboterarm nach Anspruch 1 oder 2,
bei dem die Eingabeelemente (EE) des freien Endes (FE) auf einer Fläche angeordnet sind, die gegenüber einer Fläche senkrecht zur Drehachse (DN+1) des Endeffektors (E) in einem Winkel von 5 bis 60° geneigt ist.

4. Roboterarm nach einem der Ansprüche 1 bis 3,
bei dem das freie Ende (FE) mit dem Rest des zweiten Fortsatzes (F1) in verschiedenen Ausrichtungen um die Drehachse (D_{N+1}) des Endeffektors (E) ösbar fest verbindbar ist.

5. Roboterarm nach einem der Ansprüche 1 bis 4,
bei dem das freie Ende (FE) des zweiten Fortsatzes (F1) um die Drehachse (D_{N+1}) des Endeffektors (E) rotierbar fest mit dem Rest des zweiten Fortsatzes (F1) verbunden ist.

6. Roboterarm nach einem der Ansprüche 1 bis 5,
bei dem zwischen dem freien Ende (FE) und dem Rest des zweiten Fortsatzes (F1) eine mechanische und/oder elektrische und/oder eine magnetische Rastervorrichtung vorhanden ist, die ein Rotieren des freien Endes (FE) des zweiten Fortsatzes (F1) mit einer vorgegebenen Rasterung um die Drehachse (D_{N+1}) des Endeffektors (E) erlaubt.

7. Roboter mit einem Roboterarm nach einem der Ansprüche 1 bis 6.

8. Roboter nach Anspruch 7,
mit
- Sensoren zur Erfassung eines mechanischen Zustandes (Z₍ₜ₎) des Roboterarms,
- einer Einheit zur Erfassung von Eingaben (EG₍ₜ₎) über die Eingabeelemente,
- einer Einheit zur Auswertung der Zustände (Z₍ₜ₎) und der Eingaben (EG₍ₜ₎) zur Ermittlung von Auswertergebnissen (AW₍ₜ₎) und
- eine Speichereinheit zur Speicherung der Auswerteergebnisse (AW₍ₜ₎)

9. Roboter nach Anspruch 8,
bei dem die Auswertergebnisse (AW₍ₜ₎) Steueranweisungen zur Ansteuerung des Roboterarms Roboter sind.

## Claims

1. Robotic arm having a number (N) of arm members (Aₙ) which can be connected to a robot body via the same number (N) of actuator driven articulated connections (GVₙ), with n = 1, 2, ..., N; wherein
- the distal arm member (A_{N}) of the robot arm is connected at its proximal end to the previous arm member (A_{N-1}) via the distal articulated connection (GV_{N}),
- the proximal arm member (A₁) can be connected at its proximal end to the robot body via the proximal articulated connection (GV₁),
- the distal articulated connection (GV_{N}) enables the distal arm member (A_{N}) to rotate about an axis of rotation (D_{N}),
- the distal arm member (A_{N}) of the robot arm extends along an axis (L1), which axis (L1) forms an angle of 50 to 130 ° with the axis of rotation (D_{N}) of the distal arm member (A_{N}),
- the distal end of the distal arm member (A_{N}) can be connected to an effector (E) via an additional actuator driven articulated connection (GV_{N+1}), wherein the additional articulated connection (GV_{N+1}) enables the effector (E) to rotate about an axis of rotation (D_{N+1}), wherein
- the axis of rotation (D_{N}) of the distal arm member (A_{N}) and the axis of rotation (D_{N+1}) of the end effector (E) form an angle (W1) in the range of 50 to 130 °,
- the axis of rotation (D_{N+1}) of the end effector (E) and the axis (L1) of the distal arm member (A_{N}) form an angle (W2) in the range of 50 to 130°,
- an extension (F2) that can be grasped with one hand and is rigidly connected thereto and extends perpendicularly to the axis of rotation (D_{N+1}) of the end effector (E) is designed on the effector (E) or on an intermediate piece (ZW) arranged between the articulated connection (GV_{N+1}) and the effector (E) or on the distal arm member (A_{N}), and
- the distal arm member (A_{N}) has a rigidly connected handle-like second extension (F1) which extends concentrically to the axis of rotation (D_{N+1}) of the end effector (E), wherein a free end (FE) of the second extension (F1) opposite the rest of the second extension (F1) can be rotated about the axis of rotation (D_{N+1}) of the end effector (E) or can be arranged to rotate about the axis of rotation (D_{N+1}) of the end effector, and wherein the free end (FE) of the second extension (F1) has input elements (EE) for manually inputting data.

2. Robotic arm according to Claim 1, in which one or a plurality of input elements (EE_{ZW}) are arranged on the intermediate piece (ZW) within the reach of a thumb.

3. Robotic arm according to Claim 1 or 2, in which the input elements (EE) of the free end (FE) are arranged on a surface which is inclined at an angle of 5 to 60° relative to a surface perpendicular to the axis of rotation (DN+1) of the end effector (E).

4. Robotic arm according to one of Claims 1 to 3, in which the free end (FE) can be detachably connected in a fixed manner to the rest of the second extension (F1) in different orientations about the axis of rotation (D_{N+1}) of the end effector (E).

5. Robotic arm according to one of Claims 1 to 4, in which the free end (FE) of the second extension (F1) is fixedly connected to the rest of the second extension (F1) so that it can rotate about the axis of rotation (D_{N+1}) of the end effector (E).

6. Robotic arm according to one of Claims 1 to 5, in which a mechanical and/or electrical and/or a magnetic locking device is present between the free end (FE) and the rest of the second extension (F1), allowing the free end (FE) of the second extension (F1) to rotate with predetermined locking about the axis of rotation (D_{N+1}) of the end effector (E).

7. Robot having a robotic arm according to one of Claims 1 to 6.

8. Robot according to Claim 7, having
- sensors for detecting a mechanical state (Z₍ₜ₎) of the robotic arm,
- a unit for recording inputs (EG₍ₜ₎) via the input elements,
- a unit for evaluating the states (Z₍ₜ₎) and the inputs (EG₍ₜ₎) for determining evaluation results (AW₍ₜ₎) and
- a memory unit for storing the evaluation results (AW₍ₜ₎).

9. Robot according to Claim 8, in which the evaluation results (AW₍ₜ₎) are control instructions for controlling the robotic arm of the robot.

## Revendications

1. Bras de robot, pourvu d'un nombre (N) d'éléments de bras (Aₙ), qui par l'intermédiaire du même nombre (N) d'assemblages articulés (GVₙ) pouvant être entraînés par actionneur sont susceptibles d'être assemblés avec un corps de robot, avec n = 1, 2, ..., N ;
- par son extrémité proximale, l'élément de bras (A_{N}) distal du bras de robot étant assemblé par l'intermédiaire de l'assemblage articulé (GV_{N}) distal avec l'élément de bras (A_{N-1}) précédent,
- par son extrémité proximale, l'élément de bras (A₁) proximal étant susceptible d'être assemblé par l'intermédiaire de l'assemblage articulé (GV₁) avec le corps de robot,
- l'assemblage articulé (GV_{N}) distal permettant une rotation de l'élément de bras (AN) distal autour d'un axe de rotation (D_{N}),
- l'élément de bras (A_{N}) distal du bras de robot s'étendant le long d'un axe (L₁), lequel axe (L₁) inclut avec l'axe de rotation (D_{N}) de l'élément de bras (A_{N}) distal un angle de 50 à 130 °,
- l'extrémité distale de l'élément de bras (A_{N}) distal étant susceptible d'être assemblée par l'intermédiaire d'un assemblage articulé (GV_{N+1}) supplémentaire, susceptible d'être entraîné par actionneur avec un effecteur (E), l'assemblage articulé (GV_{N+1}) supplémentaire permettant une rotation de l'effecteur (E) autour d'un axe de rotation (D_{N+1}),
- l'axe de rotation (D_{N}) de l'élément de bras (A_{N}) distal et l'axe de rotation (D_{N+1}) de l'effecteur d'extrémité (E) incluant un angle (W1) de l'ordre de 50 à 130 °,
- l'axe de rotation (D_{N+1}) de l'effecteur d'extrémité (E) et l'axe (L1) de l'élément de bras (A_{N}) distal incluant un angle (W2) de l'ordre de 50 à 130 °,
- sur l'effecteur (E) ou sur une pièce intermédiaire (ZW) placée entre l'assemblage articulé (GV_{N+1}) et l'effecteur (E) ou sur l'élément de bras (A_{N}) distal étant conçu un prolongement (F2) susceptible d'être enveloppé par une main, s'étendant à la perpendiculaire de l'axe de rotation (D_{N+1}) de l'effecteur d'extrémité (E), et donc assemblé de manière rigide et
- l'élément de bras (A_{N}) distal comportant un deuxième prolongement (F1) en forme de poignée, assemblé de manière rigide avec celui-ci, qui s'étend de manière concentrique par rapport à l'axe de rotation (D_{N+1}) de l'effecteur d'extrémité (E), une extrémité libre (FE) du deuxième prolongement (F1) étant rotative par rapport au reste du deuxième prolongement (F1) autour de l'axe de rotation (D_{N+1}) de l'effecteur d'extrémité (E) ou étant susceptible d'être placé de manière rotative autour de l'axe de rotation (D_{N+1}) de l'effecteur d'extrémité, l'extrémité libre (FE) du deuxième prolongement (F1) comportant des éléments de saisie (EE), destinés à la saisie manuelle de données.

2. Bras de robot selon la revendication 1,
sur lequel sur la pièce intermédiaire (ZW) un ou plusieurs élément(s) de saisie (EE_{ZW}) est placé à la portée d'un pouce de la main.

3. Bras de robot selon la revendication 1 ou 2,
sur lequel les éléments de saisie (EE) de l'extrémité libre (FE) sont placés sur une surface qui est inclinée d'un angle de 5 à 60 ° en rapport d'une surface perpendiculaire à l'axe de rotation (DN+1) de l'effecteur d'extrémité (E).

4. Bras de robot selon l'une quelconque des revendications 1 à 3,
sur lequel l'extrémité libre (FE) est susceptible d'être assemblée solidement de manière amovible avec le reste du deuxième prolongement (F1) dans différentes orientations autour de l'axe de rotation (D_{N+1}) de l'effecteur d'extrémité (E).

5. Bras de robot selon l'une quelconque des revendications 1 à 4,
sur lequel l'extrémité libre (FE) du deuxième prolongement (F1) est assemblée solidement avec le reste du deuxième prolongement (F1) en étant rotative autour de l'axe de rotation (D_{N+1}) de l'effecteur d'extrémité (E).

6. Bras de robot selon l'une quelconque des revendications 1 à 5,
sur lequel entre l'extrémité libre (FE) et le reste du deuxième prolongement (F1) est présent un dispositif d'enclenchement mécanique et/ou électrique et/ou magnétique, qui admet une rotation de l'extrémité libre (FE) du deuxième prolongement (F1) avec un crantage prédéfini autour de l'axe de rotation (D_{N+1}) de l'effecteur final (E).

7. Robot, pourvu d'un bras de robot selon l'une quelconque des revendications 1 à 6.

8. Robot selon la revendication 7,
pourvu
- de capteurs destinés à détecter un état mécanique (Z₍ₜ₎) du bras de robot,
- d'une unité destinée à saisir des données (EG₍ₜ₎) par l'intermédiaire des éléments de saisie,
- d'une unité destinée à évaluer les états (Z₍ₜ₎) et les saisies (EG₍ₜ₎), pour déterminer des résultats d'évaluation (AW₍ₜ₎) et
- d'une unité de mémorisation destinée à mémoriser les résultats d'évaluation (AW₍ₜ₎).

9. Robot selon la revendication 8,
sur lequel les résultats d'évaluation (AW₍ₜ₎) sont des instructions de commande pour l'activation du bras de robot du robot.
